# EUROPEAN PATENT APPLICATION

(11) **EP 4 796 111 A2**
(43) Date of publication of application: **26.08.2026**
(21) Application number: 26191604.3
(22) Date of filing: 02.03.2022
(51) Int. Cl.: A61K 31/155

(54) **STABLE PHARMACEUTICAL COMPOSITIONS OF METFORMIN**

(30) Priority: 03.03.2021 FI 20215229
(62) Document number(s) of the earlier application(s) in accordance with Art. 76 EPC: 22709764.9 / 4 301 346
(71) Applicant: ORION CORPORATION, 02200 Espoo (FI)
(72) Inventor: HAAJANTO, Tapio, 02101 Espoo (FI); PARTANEN, Marja, 02101 Espoo (FI); SALMIA, Jukka, 02101 Espoo (FI)
(74) Representative: J A Kemp LLP

(57) **Abstract**

The invention relates to pharmaceutical compositions, particularly tablets, comprising metformin or a pharmaceutically acceptable salt thereof as an active ingredient together with at least one excipient and an inhibitor of peroxide-induced oxidation. The compositions show improved chemical stability against formation of potentially carcinogenic nitrosamine compounds such as N-nitrosodimethylamine (NDMA).

## Description

### Technical field

The present invention relates to pharmaceutical compositions, particularly tablets, comprising metformin or a pharmaceutically acceptable salt thereof as an active ingredient. The compositions show improved chemical stability against formation of potentially carcinogenic nitrosamine compounds such as N-nitrosodimethylamine (NDMA).

### Background of the invention

Metformin is a commonly used anti-diabetic drug, which is the preferred initial medication for most of the patients with type 2 diabetes mellitus. Metformin has a relatively high allowed dosage up to 2-3 g/day in Europe and the USA. Recent detection of excess amounts of potentially carcinogenic nitrosamine impurities, particularly N-nitrosodimethylamine (NDMA), in metformin dosage forms, has created concern among health care providers and patients, and has caused recall of many metformin dosage forms, particularly extended release tablets. Recommended maximum allowed limit of NDMA in metformin products is set to 0.032 ppm by the European Medicines Agency. The root cause of NDMA source in metformin products has been investigated but definitive cause has remained unsolved.

Thus, there is a need for metformin compositions, which would avoid the problem of nitrosamine impurities in the dosage forms.

### Summary of the invention

It has been found that the formation nitrosamines such as NDMA in metformin products can be reduced by introducing inhibitors of peroxide-induced oxidation such as citric acid or ascorbic acid into the metformin formulations. It was also found that peroxides are commonly present as impurities in excipient materials typically used in the preparation of metformin products. While not wishing to be bound by theory, it is believed that the formation of nitrosamines is caused by these peroxide impurities reacting with metformin during the manufacture and storage of the metformin products. The reactivity of peroxides can be reduced by inhibitors of peroxide-induced oxidation leading to reduced formation of nitrosamines such as NDMA in metformin products.

Thus, according to one embodiment of the invention, the present invention provides the use of an inhibitor of peroxide-induced oxidation for reducing the formation nitrosamines, particularly NDMA, in a pharmaceutical composition, which comprises metformin or a pharmaceutically acceptable salt thereof and at least one excipient.

According to another embodiment, the present invention provides a pharmaceutical composition comprising metformin or a pharmaceutically acceptable salt thereof, at least one excipient and from about 0.01 to about 10 %, preferably from about 0.05 to about 5 %, typically from about 0.1 to about 2 %, for example from about 0.25 to about 1 %, of an inhibitor of peroxide-induced oxidation, per total weight of excipients.

According to another embodiment, the present invention provides a method for preparing a pharmaceutical composition comprising metformin or a pharmaceutically acceptable salt thereof and at least one excipient, which comprises contacting the at least one excipient with an amount of an inhibitor of peroxide-induced oxidation effective to reduce the formation nitrosamines in the composition.

### Detailed description of the invention

The term "ppm", as used herein, refers to parts per million per weight. Thus, 1 ppm per weight corresponds to 1 mg/kg.

The term "inhibitor of peroxide-induced oxidation", as used herein, refers to a group of substances that are capable of inhibiting peroxide-induced degradation reactions of metformin. The inhibition can take place, for example, by eliminating or reducing peroxides or by inhibiting, preventing or reducing oxidative reactions of peroxides. Examples of inhibitors of peroxide-induced oxidation include "antioxidants" and "stabilizing acids", as referred to below.

The term "antioxidant", as used herein, refers to a group of substances that are capable of inhibiting, preventing or reducing oxidative reactions. Preferred are water-soluble antioxidants, examples of which include ascorbic acid, acetyl cysteine, cysteine, thiourea, 1-thiosorbitol, sulphites (e.g. sodium sulfite), bisulfites (e.g. sodium bisulfite), metabisulfites (e.g. sodium metabisulfite), thiosulfates (e.g. sodium thiosulfate) or pharmaceutically acceptable salts thereof.

The term "stabilizing acid", as used herein, refers to a pharmaceutically acceptable acid having pKa lower than 4.7, for example from about -8 to lower than 4.7, from about -2 to about 4.5, or from about 1 to about 4.5, and being capable of stabilizing metformin against peroxide induced reactions in pharmaceutical compositions, particularly tablets. Examples of stabilizing acids include citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid and hydrochloric acid.

The term "nitrosamine", as used herein, refers to an organic compound of the chemical structure R₁(R₂)N-N=O, wherein R₁ and R₂ is an alkyl or a derivative of an alkyl group. Examples of "nitrosamine" include *N*-nitrosodimethylamine (NDMA), *N*-nitrosodiethylamine (NDEA) and *N*-nitroso-N-methyl-4-aminobutyric acid (NMBA). Nitrosamine impurities may increase the risk of cancer in people exposed to them above acceptable levels and over long periods.

The term "cellulose derivative", as used herein, refers to cellulose ether derivatives commonly used as excipients in pharmaceutical dosage forms such as tablets. Examples of "cellulose derivatives" include methylcellulose (MC), ethyl cellulose (EC), hydroxyethyl cellulose (HEC), hydroxypropyl cellulose (HPC), hydroxypropylmethyl cellulose (HPMC), carboxymethyl cellulose (CMC) and sodium carboxymethyl cellulose (NaCMC).

The term "methacrylate polymer", as used herein, refers to a polymer, usually a copolymer, prepared from a methacrylate or methyl methacrylate monomer. Examples of methacrylate polymers include EUDRAGIT^{®} L100-55 which is a copolymer of methacrylic acid and ethyl acrylate and EUDRAGIT^{®} L100 which is a copolymer of methacrylic acid and methyl methacrylate and EUDRAGIT^{®} RS 100 which is a copolymer of ethyl acrylate, methyl methacrylate and a low content of methacrylic acid ester with quaternary ammonium groups.

The term "polyvinylpyrrolidone derivative", as used herein, refers to polyvinylpyrrolidone (also called povidone or PVP), copolymers of 1-vinyl-2-pyrrolidone such as copovidone (copolymer of 1-vinyl-2-pyrrolidone and vinyl acetate) and cross-linked polyvinylpyrrolidone (crospovidone).

The term "polysorbate", as used herein, refers to oleate esters of sorbitol and its anhydrides, typically copolymerized with ethylene oxide. Preferred polysorbates are Polysorbate 20 (poly(ethylene oxide) (20) sorbitan monolaurate, Tween 20) or Polysorbate 80 (poly(ethylene oxide) (80) sorbitan monolaurate, Tween 80).

The term "polyethylene glycol", as used herein, refers to a polymer containing ethylene glycol monomer units of formula -O-CH₂-CH₂-. Polyethylene glycols (also known as Macrogols) may have average molecular weight from about 200 to about 9000 g/mol, from about 200 to about 5000 g/mol or from about 200 to about 900 g/mol. Examples of polyethylene glycol include polyethylene glycols include PEG 200, PEG 300, PEG 400 and PEG 1000.

The term "water soluble", as used herein, refers to water solubility of 10 mg/ ml or higher, preferably 20 mg/ml or higher, more preferably 50 mg/ml or higher.

The present invention relates to the use of an inhibitor of peroxide-induced oxidation for reducing the formation nitrosamines, particularly NDMA, in a pharmaceutical composition which comprises metformin or a pharmaceutically acceptable salt thereof and at least one excipient.

Particularly suitable inhibitors of peroxide-induced oxidation are antioxidants and stabilizing acids.

Particularly suitable antioxidants are water-soluble antioxidants such as ascorbic acid, acetyl cysteine, cysteine, thiourea, 1-thiosorbitol, sulphites (e.g. sodium sulfite), bisulfites (e.g. sodium bisulfite), metabisulfites (e.g. sodium metabisulfite), thiosulfates (e.g. sodium thiosulfate) and pharmaceutically acceptable salts thereof. Preferred are ascorbic acid, sulfites, bisulfites and metabisulfites and pharmaceutically acceptable salts thereof, for example sodium salts such as sodium ascorbate, sodium sulfite, sodium bisulfite and sodium metabisulfite. Particularly preferred is ascorbic acid.

Suitable stabilizing acids are pharmaceutically acceptable acids having pKa lower than 4.7, for example from about -8 to lower than 4.7, from about -2 to about 4.5, or from about 1 to about 4.5. Particularly suitable stabilizing acids include, for example, citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid and hydrochloric acid. Preferred are citric acid, tartaric acid, phosphoric acid, malic acid and malonic acid. Citric acid is particularly preferred as it is edible component and not considered active ingredient in oral pharmaceutical products.

Inhibitors of peroxide-induced oxidation are suitably used in metformin dosage forms, which comprise pharmaceutical excipients containing peroxide impurities. According to one aspect of the invention, such metformin dosage form is a tablet, particularly a prolonged release tablet. Typically, in humans a prolonged release tablet of metformin hydrochloride exhibits Tₘₐₓ (the time to reach maximum concentration in plasma) at the time which is at least about 4 hours, more typically at least about 5 hours, for example about 6-7 hours, after ingestion.

Pharmaceutical excipients which are particularly used in tablets and which have been found to contain peroxides include, but are not limited to, cellulose derivatives, methacrylate polymers, polyvinylpyrrolidone derivatives, polysorbates and polyethylene glycols.

Typical cellulose derivatives used in tablets include, but are not limited to, methylcellulose (MC), ethyl cellulose (EC), hydroxyethyl cellulose (HEC), hydroxypropyl cellulose (HPC), hydroxypropylmethyl cellulose (HPMC), carboxymethyl cellulose (CMC) and sodium carboxymethyl cellulose (NaCMC). HPMC and HPC are particular examples of cellulose derivatives which commonly contains peroxides.

Typical methacrylate polymers used in tablets include, but are not limited to, a copolymer of methacrylic acid and ethyl acrylate (available e.g. under the trade name EUDRAGIT^{®} L100-55), a copolymer of methacrylic acid and ethyl acrylate (available e.g. under the trade name EUDRAGIT^{®} L100), and a copolymer of ethyl acrylate, methyl methacrylate and a low content of methacrylic acid ester with quaternary ammonium groups (available e.g. under the trade name EUDRAGIT^{®} RS 100).

Typical polyvinylpyrrolidone derivatives used in tablets include, but are not limited to, polyvinylpyrrolidone (PVP, also known as povidone), copovidone (copolymer of 1-vinyl-2-pyrrolidone and vinyl acetate) and cross-linked polyvinylpyrrolidone (crospovidone). PVP is a particular example of a polyvinylpyrrolidone derivative which commonly contains peroxides.

Typical polysorbates used in tablets include, but are not limited to, Polysorbate 20 (poly(ethylene oxide) (20) sorbitan monolaurate) and Polysorbate 80 (poly(ethylene oxide) (80) sorbitan monolaurate, also known as Tween 80).

Typical polyethylene glycols (also known as Macrogols) used in tablets include, but are not limited to, PEG 200, PEG 300, PEG 400, PEG 1000 and PEG 6000.

According to one aspect, the present invention discloses a pharmaceutical composition comprising metformin or a pharmaceutically acceptable salt thereof, at least one excipient and from about 0.01 to about 10 %, preferably from about 0.05 to about 5 %, typically from about 0.1 to about 2 %, for example from about 0.25 to about 1 %, of an inhibitor of peroxide-induced oxidation, per total weight of excipients.

Preferred inhibitors of peroxide-induced oxidation to be used in the above pharmaceutical composition are antioxidants, particularly water-soluble antioxidants, and stabilizing acids. Suitable water-soluble antioxidants include ascorbic acid, acetyl cysteine, cysteine, thiourea, 1-thiosorbitol, sulphites (e.g. sodium sulfite), bisulfites (e.g. sodium bisulfite), metabisulfites (e.g. sodium metabisulfite), thiosulfates (e.g. sodium thiosulfate) or pharmaceutically acceptable salts thereof. Preferred are ascorbic acid, sulfites, bisulfites and metabisulfites and pharmaceutically acceptable salts thereof, for example sodium salts such as sodium ascorbate, sodium sulfite, sodium bisulfite and sodium metabisulfite. Ascorbic acid is particularly preferred. Examples of suitable stabilizing acids include citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid and hydrochloric acid. Preferred are citric acid, tartaric acid, phosphoric acid, malic acid and malonic acid. Citric acid being particularly preferred. In one aspect, the inhibitor of peroxide-induced oxidation is citric acid or ascorbic acid.

According to one embodiment, the present invention discloses a pharmaceutical composition comprising
(a) 40 - 90 %, preferably 50 to 80 %, for example 55 - 75 %, per weight of the composition, of metformin or a pharmaceutically acceptable salt thereof,
(b) 0.01 - 5 %, preferably 0.02 - 2 %, for example 0.05 - 1 %, for example 0.1 - 0.5 %, per weight of the composition, of an inhibitor of peroxide-induced oxidation;
(c) 5 - 60 %, preferably, 10 - 50 %, for example 20 - 40 %, per weight of the composition, of at least one excipient.

According to one aspect of the above embodiment, the excipient comprises at least one excipient selected from the group consisting of cellulose derivatives, methacrylate polymers, polyvinylpyrrolidone derivatives, polysorbates and polyethylene glycols. According to another aspect, the excipient comprises at least one excipient selected from the group consisting of cellulose derivatives, methacrylate polymers and polyvinylpyrrolidone derivatives. According to another aspect, the excipient comprises at least one excipient selected from the group consisting of HPMC, HPC, povidone and copovidone. According to another aspect, the excipient comprises at least one excipient selected from the group consisting of HPMC, povidone and copovidone. According to another aspect, the excipient comprises at least one excipient selected from the group consisting of HPMC and copovidone. According to another aspect, the excipient is HPMC.

According to another aspect of the above embodiment, the inhibitor of peroxide-induced oxidation is an antioxidant, particularly a water-soluble antioxidant, or a stabilizing acid. According to one aspect, the water-soluble antioxidant is ascorbic acid, a sulfite, a bisulfite, a metabisulfite or a pharmaceutically acceptable salt thereof, for example, a sodium salt such as sodium ascorbate, sodium sulfite, sodium bisulfite or sodium metabisulfite. In one aspect, the antioxidant is ascorbic acid.

According to another aspect, the stabilizing acid in the above embodiment is citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid or hydrochloric acid. In one aspect, the stabilizing acid is citric acid, tartaric acid, phosphoric acid, malic acid or malonic acid. In one aspect, the stabilizing acid is citric acid.

In still another aspect, the inhibitor of peroxide-induced oxidation is citric acid or ascorbic acid.

According to still another embodiment, the present invention discloses a pharmaceutical composition, particularly a prolonged release tablet, comprising
(a) 40 - 90 %, preferably 50 to 80 %, for example 55 - 75 %, per weight of the composition, of metformin or a pharmaceutically acceptable salt thereof,
(b) 0.01 - 5 %, preferably 0.02 - 2 %, for example 0.05 - 1 %, for example 0.1 - 0.5 %, per weight of the composition, of an inhibitor of peroxide-induced oxidation;
(c) 0.5 - 10 %, preferably 1 - 8 %, for example 2 - 5 %, per weight of the composition, of a binder;
(d) 5 - 50 %, preferably 10 - 45 %, for example 15 - 40 %, per weight of the composition, of a release controlling polymer selected from cellulose derivatives and methacrylate polymers; and
(e) 0.1 - 5 %, preferably 0.2 - 2 %, for example 0.3 - 1 %, per weight of the composition, of lubricant selected from magnesium stearate and stearic acid.

According to one aspect of the above embodiment, the inhibitor of peroxide-induced oxidation is a water-soluble antioxidant or a stabilizing acid. According to another aspect, the water-soluble antioxidant is ascorbic acid, a sulfite, a bisulfite, a metabisulfite or a pharmaceutically acceptable salt thereof, for example a sodium salt such as sodium ascorbate, sodium sulfite, sodium bisulfite or sodium metabisulfite. In one aspect, the antioxidant is ascorbic acid.

According to another aspect, the stabilizing acid is citric acid, tartaric acid, phosphoric acid, malic acid, lactic acid, methanesulfonic acid, ethanesulfonic acid, *p-*toluenesulfonic acid, oxalic acid, malonic acid, succinic acid, glutaric acid, adipic acid, gluconic acid, glucuronic acid, glutamic acid, fumaric acid, maleic acid, hyaluronic acid or hydrochloric acid. In one aspect, the stabilizing acid is citric acid, tartaric acid, phosphoric acid, malic acid or malonic acid. In one aspect, the stabilizing acid is citric acid. In still another aspect, the inhibitor of peroxide-induced oxidation is citric acid or ascorbic acid. In one aspect, the binder is povidone (PVP) or copovidone. In one aspect, the binder is povidone (PVP). In another aspect, the binder is copovidone. In one aspect, the release-controlling polymer is HPMC or HPC. In one aspect, the release-controlling polymer is HPMC. In another aspect, the release-controlling polymer is HPC.

The composition according to the present invention is suitably in the form of a tablet, for example intended for immediate or prolonged release of the active ingredient. Preferably, the composition is in the form of a prolonged release tablet. Typically, in humans a prolonged release tablet of metformin hydrochloride exhibits Tₘₐₓ (the time to reach maximum concentration in plasma) at the time which is at least about 4 hours, more typically at least about 5 hours, for example about 6-7 hours, after ingestion.

According to one embodiment of the invention, the composition may comprise in addition to metformin or a pharmaceutically acceptable salt thereof one or more other active ingredient(s) useful, for example, in the treatment of diabetes mellitus.

According to another embodiment of the invention, the composition according to the invention comprises metformin or a pharmaceutically acceptable salt thereof as a sole active ingredient.

According to one embodiment of the invention, the composition according to the invention is not an effervescent or a gastric floating composition, particularly a composition which comprises an acid, for example citric acid, together with a base, for example NaHCO₃, for the release of carbon dioxide.

The preparation of the composition comprises contacting at least one excipient with an amount of an inhibitor of peroxide-induced oxidation effective to reduce the formation nitrosamines in the composition.

The tablet composition can be prepared by known methods, for example by wet granulation, dry granulation or dry compression. According to one preferred aspect of the invention, the tablet composition is prepared by wet granulation comprising water as granulating liquid.

The method of preparing the tablet by wet granulation generally comprises the steps of
(a) dissolving the inhibitor of peroxide-induced oxidation in a granulating liquid comprising water;
(b) granulating the mixture comprising at least one excipient with the granulating liquid of step (a);
(c) drying the wet granules;
(d) optionally mixing the granules with further excipients; and
(e) compressing the resulting mass into tablets; and, optionally, coating the tablet with one or further pharmaceutically acceptable film-coating agent.

The mixture of step (b) may also comprise metformin or a pharmaceutically acceptable salt thereof. Alternatively, step (c) can be followed by a further step of mixing metformin or a pharmaceutically acceptable salt thereof with the granules obtained in step (c) followed by further granulation of the resulting mixture with a granulating liquid comprising water; and drying the resulting granules.

The wet granulated prolonged release tablet is suitably prepared, for example, by first mixing metformin or a pharmaceutically acceptable salt thereof, the binder (for example povidone or copovidone) and a release controlling polymer (for example HPMC or HPC), optionally with other excipients, in a suitable granulator vessel, for example high shear granulator. The inhibitor of peroxide-induced oxidation (for example citric acid or ascorbic acid) is then dissolved in granulating liquid, preferably water, for obtaining a granulating liquid. The above mixture is then granulated by spaying the granulating liquid in a suitable vessel, for example a fluidized bed granulator. The obtained granules are dried and screened, for example, using a screening mill unit, optionally together with further excipients (for example a glidant such as fumed silica). Lubricant (for example magnesium stearate or stearic acid) can then be added to the mass of the previous step followed by blending. The tablet mass can then be compressed into tablet cores, for example, in a power assisted rotary tablet press.

The tablet cores can be provided with a water-soluble film coating, if desired, to facilitate tablet swallowing, to protect from direct contact with the drug substance and to improve aesthetics. Suitable film coating agents can be selected from the group of plasticizers, film-forming agents and colorants. Optionally an anti-tacking agent or opacifier can be used. The plasticizer, such as polyethylene glycol (PEG), the film-forming agent, such as hydroxypropylmethyl cellulose (HPMC), and the colorants, such as ferric oxide and titanium dioxide, are combined with film-coating liquids, preferably water, to result in a homogeneous coating suspension which is brought up, preferably sprayed, on the tablets in a suitable coating device, such as for example a perforated drum coater.

Metformin or a pharmaceutically acceptable salt thereof, particularly hydrochloride salt, is suitably administered in an amount ranging from about 500 mg to about 3000 mg, per day to the patient, for the treatment of type 2 diabetes mellitus. The dose can be administered once daily or divided to several times a day, for example twice daily. The composition of the invention, such as a tablet, may comprise metformin or a pharmaceutically acceptable salt thereof, particularly hydrochloride salt, in an amount ranging from about 200 mg to about 2000 mg, typically from about 500 mg to about 1000 mg, for example 500 mg, 750 mg or 1000 mg of metformin hydrochloride. Such composition can be administered once or several times a day, for example one tablet or several tablets once, twice or several times daily.

The invention is further illustrated by the following examples, which are not meant to limit the scope of the invention.

Example 1. Preparation of prolonged release metformin HCl tablets

Metformin prolonged release tablet formulations A - E were prepared. Formulations A and E were prepared by dry mixing metformin HCl, hydroxypropyl methylcellulose (HPMC K4M) and copovidone or low substituted hydroxypropylcellulose (L-HPC LH-21) followed by wet granulation of the mixture by spraying water to the mixture. The formed granules were dried. The dry granules were mixed with fumed silica (Aerosil 200) and magnesium stearate to produce a tablet mass which was finally compressed into tablets. Formulations B-D were prepared similarly except that the mixture of HPMC and copovidone was first treated by spraying the mixture with a water solution (10 %) of citric and/or ascorbic acid followed by drying the mixture. The final formulations of each tablets mass A-E are shown in Table 1. From each tablet mass was compressed tablets with total weight of about 460 mg.

**Table 1. Tested formulations**

| **Material** | **A (%)** | **B (%)** | **C (%)** | **D (%)** | **E (%)** |
|---|---|---|---|---|---|
| **Intragranular part** | | | | | |
| Metformin HCl | 65.8 | 64.7 | 64.7 | 64.8 | 65.8 |
| HPMC K4M | 28.9 | 28.5 | 28.5 | 28.5 | 28.9 |
| Copovidone | 2.9 | 2.8 | 2.8 | 2.8 | - |
| Citric acid | - | 1.6 | - | 0.9 | - |
| Ascorbic acid | - | - | 1.6 | 0.6 | - |
| L-HPC LH-21 | - | - | - | - | 2.9 |

| **Extragranular part** | | | | | |
|---|---|---|---|---|---|
| Fumed silica (Aerosil 200) | 1.9 | 1.9 | 1.9 | 1.9 | 1.9 |
| Magnesium stearate | 0.5 | 0.5 | 0.5 | 0.5 | 0.5 |
| **Total** | 100.0 | 100.0 | 100.0 | 100.0 | 100.0 |

Example 2. Stability of the extended release metformin HCl tablets against NDMA formation

The tablet mass and the compressed tablets of Example 1 were put to the stressed stability test for 4 days at 60 °C. The initial amount of NDMA in the tablet mass and compressed tablets and after stressing for 4 days were determined. The results are shown in Tables 2 and 3. In the Tables, 0 ppm means NDMA level under the detection limit (0.003 ppm). The maximum allowed limit for NDMA in metformin products currently set by the European Medicines Agency is 0.032 ppm.

**Table 2. Formation of NDMA in the tablet mass**

| | **Formulation** | **NDMA on Day 0 (ppm)** | **NDMA on Day 4 (ppm)** |
|---|---|---|---|
| **Tablet mass** | A | 0.022 | 0.075 |
| | B | 0 | 0 |
| | C | 0 | 0 |
| | D | 0 | 0 |
| | E | 0.025 | 0.092 |

**Table 3. Formation of NDMA in the pressed tablets**

| | **Formulation** | **NDMA on Day 0 (ppm)** | **NDMA on Day 4 (ppm)** |
|---|---|---|---|
| **Tablet** | A | 0.019 | 0.059 |
| | B | 0.016 | 0 |
| | C | 0 | 0 |
| | D | 0 | 0 |
| | E | 0.019 | 0.086 |

It can be seen that using 5 % of citric or ascorbic acid, per total weight of the granule excipients, results in significantly reduced NDMA formation during manufacture of the tablet mass and also during the storage of the tablets in the stressed conditions. The experiment was repeated using 0.5 %, 1.5 % and 3 % of citric or ascorbic acid, per total weight of the granule excipients and similar reduction of NDMA formation was observed.

Aspects of the disclosure
1. Use of an inhibitor of peroxide-induced oxidation for reducing the formation nitrosamines in a pharmaceutical composition which comprises metformin or a pharmaceutically acceptable salt thereof and at least one excipient.
2. Use according to aspect 1, wherein the inhibitor of peroxide-induced oxidation is an antioxidant or a stabilizing acid.
3. Use according to aspect 2, wherein the antioxidant is a water-soluble antioxidant.
4. Use according to aspect 3, wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof.
5. Use according to any of aspects 2 to 4, wherein the stabilizing acid is citric acid, tartaric acid, phosphoric acid, malic acid, lactic acid, methanesulfonic acid, ethanesulfonic acid, p-toluenesulfonic acid, oxalic acid, malonic acid, succinic acid, glutaric acid, adipic acid, gluconic acid, glucuronic acid, glutamic acid, fumaric acid, maleic acid, hyaluronic acid or hydrochloric acid.
6. Use according to aspect 1 or 2, wherein the inhibitor of peroxide-induced oxidation is citric acid or ascorbic acid.
7. Use according to any one of aspects 1 to 6, wherein the excipient is selected from the group consisting of cellulose derivatives, methacrylate polymers, polyvinylpyrrolidone derivatives, polysorbates and polyethylene glycols.
8. Use according to any one of aspects 1 to 7, wherein the composition is a prolonged release tablet.
9. A pharmaceutical composition comprising metformin or a pharmaceutically acceptable salt thereof, at least one excipient and from about 0.01 to about 10 %, preferably from about 0.05 to about 5 %, more preferably from about 0.1 to about 2 %, of an inhibitor of peroxide-induced oxidation, per total weight of excipients.
10. A composition according to aspect 9, wherein the inhibitor of peroxide-induced oxidation is an antioxidant or a stabilizing acid.
11. A composition according to aspect 10, wherein the antioxidant is a water-soluble antioxidant.
12. A composition according to aspect 11, wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof.
13. A composition according to any of aspects 10 to 12, wherein the stabilizing acid is citric acid, tartaric acid, phosphoric acid, malic acid, lactic acid, methanesulfonic acid, ethanesulfonic acid, p-toluenesulfonic acid, oxalic acid, malonic acid, succinic acid, glutaric acid, adipic acid, gluconic acid, glucuronic acid, glutamic acid, fumaric acid, maleic acid, hyaluronic acid or a hydrochloric acid.
14. A composition according to aspect 9 or 10, wherein the inhibitor of peroxide-induced oxidation is citric acid or ascorbic acid.
15. A composition according to any one of aspects 9 to 14, wherein the excipient is selected from the group consisting of cellulose derivatives, methacrylate polymers, polyvinylpyrrolidone derivatives, polysorbates and polyethylene glycols.
16. A composition according to any one of aspects 9 to 15, wherein the composition is a prolonged release tablet.
17. A method for preparing a pharmaceutical composition comprising metformin or a pharmaceutically acceptable salt thereof and at least one excipient, which comprises contacting the at least one excipient with an amount of an inhibitor of peroxide-induced oxidation effective to reduce the formation nitrosamines in the composition.
18. A method according to aspect 17, wherein the inhibitor of peroxide-induced oxidation is an antioxidant or a stabilizing acid.
19. A method according to aspect 18, wherein the antioxidant is a water-soluble antioxidant.
20. A method according to aspect 19, wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof.
21. A method according to any of aspects 17 to 20, wherein the stabilizing acid is citric acid, tartaric acid, phosphoric acid, malic acid, lactic acid, methanesulfonic acid, ethanesulfonic acid, p-toluenesulfonic acid, oxalic acid, malonic acid, succinic acid, glutaric acid, adipic acid, gluconic acid, glucuronic acid, glutamic acid, fumaric acid, maleic acid, hyaluronic acid or a hydrochloric acid.
22. A method according to aspect 17 or 18, wherein the inhibitor of peroxide-induced oxidation is citric acid or ascorbic acid.
23. A method according to any one of aspects 17 to 22, wherein the excipient is selected from the group consisting of cellulose derivatives, methacrylate polymers, polyvinylpyrrolidone derivatives, polysorbates and polyethylene glycols.
24. A method according to any one of aspects 17 to 23, wherein the composition is a prolonged release tablet.
25. A method according to any of aspects 17 to 24, comprising the steps of
   (a) dissolving the inhibitor of peroxide-induced oxidation in a granulating liquid comprising water;
   (b) granulating the mixture comprising at least one excipient with the granulating liquid of step (a);
   (c) drying the wet granules;
   (d) optionally mixing the granules with further excipients; and
   (d) compressing the resulting mass into tablets; and, optionally, coating the tablet with one or further pharmaceutically acceptable film-coating agent.
26. A method according to aspect 25, wherein the mixture of step (b) also comprises metformin or a pharmaceutically acceptable salt thereof.
27. A method according to aspect 25, wherein step (c) is followed by a further step of mixing metformin or a pharmaceutically acceptable salt thereof with the granules obtained in step (c) followed by further granulation of the resulting mixture with a granulating liquid comprising water; and drying the resulting granules.

## Claims

1. Use of an inhibitor of peroxide-induced oxidation for reducing the formation nitrosamines in a pharmaceutical composition which comprises metformin or a pharmaceutically acceptable salt thereof and at least one excipient.

2. Use according to claim 1, wherein the inhibitor of peroxide-induced oxidation is:
(a) an antioxidant or a stabilizing acid, wherein, preferably:
(i) the antioxidant is a water-soluble antioxidant, further preferably wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof; and/or
(ii) the stabilizing acid is citric acid, tartaric acid, phosphoric acid, malic acid, lactic acid, methanesulfonic acid, ethanesulfonic acid, p-toluenesulfonic acid, oxalic acid, malonic acid, succinic acid, glutaric acid, adipic acid, gluconic acid, glucuronic acid, glutamic acid, fumaric acid, maleic acid, hyaluronic acid or hydrochloric acid; or
(b) citric acid or ascorbic acid.

3. Use according to claim 1 or claim 2, wherein the excipient is selected from the group consisting of cellulose derivatives, methacrylate polymers, polyvinylpyrrolidone derivatives, polysorbates and polyethylene glycols.

4. Use according to any one of claims 1 to 3, wherein the composition is a prolonged release tablet.

5. A pharmaceutical composition comprising metformin or a pharmaceutically acceptable salt thereof, at least one excipient and from about 0.01 to about 10 %, preferably from about 0.05 to about 5 %, more preferably from about 0.1 to about 2 %, of an inhibitor of peroxide-induced oxidation, per total weight of excipients.

6. A composition according to claim 5, wherein the inhibitor of peroxide-induced oxidation is:
(a) an antioxidant or a stabilizing acid, wherein, preferably:
(i) the antioxidant is a water-soluble antioxidant, further preferably wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof; and/or
(ii) the stabilizing acid is citric acid, tartaric acid, phosphoric acid, malic acid, lactic acid, methanesulfonic acid, ethanesulfonic acid, p-toluenesulfonic acid, oxalic acid, malonic acid, succinic acid, glutaric acid, adipic acid, gluconic acid, glucuronic acid, glutamic acid, fumaric acid, maleic acid, hyaluronic acid or a hydrochloric acid; or
(b) citric acid or ascorbic acid.

7. A composition according to claim 5 or claim 6, wherein the excipient is selected from the group consisting of cellulose derivatives, methacrylate polymers, polyvinylpyrrolidone derivatives, polysorbates and polyethylene glycols.

8. A composition according to any one of claims 5 to 7, wherein the composition is a prolonged release tablet.

9. A method for preparing a pharmaceutical composition comprising metformin or a pharmaceutically acceptable salt thereof and at least one excipient, which comprises contacting the at least one excipient with an amount of an inhibitor of peroxide-induced oxidation effective to reduce the formation nitrosamines in the composition.

10. A method according to claim 9, wherein the inhibitor of peroxide-induced oxidation is:
(a) an antioxidant or a stabilizing acid, wherein, preferably:
(i) the antioxidant is a water-soluble antioxidant, further preferably wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof; and/or
(ii) the stabilizing acid is citric acid, tartaric acid, phosphoric acid, malic acid, lactic acid, methanesulfonic acid, ethanesulfonic acid, *p-*toluenesulfonic acid, oxalic acid, malonic acid, succinic acid, glutaric acid, adipic acid, gluconic acid, glucuronic acid, glutamic acid, fumaric acid, maleic acid, hyaluronic acid or a hydrochloric acid; or
(b) citric acid or ascorbic acid.

11. A method according to claim 9 or claim 10, wherein the excipient is selected from the group consisting of cellulose derivatives, methacrylate polymers, polyvinylpyrrolidone derivatives, polysorbates and polyethylene glycols.

12. A method according to any one of claims 9 to 11, wherein the composition is a prolonged release tablet.

13. A method according to any of claims 9 to 12, comprising the steps of
(a) dissolving the inhibitor of peroxide-induced oxidation in a granulating liquid comprising water;
(b) granulating the mixture comprising at least one excipient with the granulating liquid of step (a);
(c) drying the wet granules;
(d) optionally mixing the granules with further excipients; and
(d) compressing the resulting mass into tablets; and, optionally, coating the tablet with one or further pharmaceutically acceptable film-coating agent.

14. A method according to claim 13, wherein the mixture of step (b) also comprises metformin or a pharmaceutically acceptable salt thereof.

15. A method according to claim 13, wherein step (c) is followed by a further step of mixing metformin or a pharmaceutically acceptable salt thereof with the granules obtained in step (c) followed by further granulation of the resulting mixture with a granulating liquid comprising water; and drying the resulting granules.
